Europäisches Patentamt

European Patent Office

Office européen des brevets

(11) Publication number: **0 265 207**
**A1**

(12) **EUROPEAN PATENT APPLICATION**

(21) Application number: **87309214.2**

(22) Date of filing: **19.10.87**

(51) Int. Cl.⁴: **A 61 F 2/48**

(30) Priority: **20.10.86 US 920432**

(43) Date of publication of application:
**27.04.88 Bulletin 88/17**

(84) Designated Contracting States:
**AT BE CH DE ES FR GB GR IT LI LU NL SE**

(71) Applicant: **VANCE PRODUCTS INCORPORATED**
**1100 West Morgan Street**
**Spencer Indiana 47460 (US)**

(72) Inventor: **French, Gerald J.**
**P.O. Box 261**
**Spencer Indiana 47460 (US)**

**Laster, Robert J., Jr.**
**R. 1, Box 436**
**Spencer Indiana 47460 (US)**

**McGough, Jeffrey D.**
**R. 1,**
**Spencer Indiana 47460 (US)**

**Stines, Joseph R.**
**R. 1, Box 379-A**
**Spencer Indiana 47868 (US)**

(74) Representative: **Bannerman, David Gardner et al**
**Withers & Rogers 4 Dyer's Buildings Holborn**
**London, EC1N 2JT (GB)**

(54) Trans-urethral incontinence device.

(57) This invention describes a urethra insertable in-dwelling incontinence control device. In a typical embodiment of this invention, a flexible tube (10) has two deformable retention means (12, (13) affixed to the exterior of the tube near one end, and a valve (20) bonded to the tube at the opposite end. Each of the deformable retention means (12, (13) is composed of a foam cuff (13a) having interconnecting interstices filled with air when the cuff is (13a) in its normally expanded condition. A lumen (11) extending along the length of the interior surface of the flexible tube and plugged at each end communicates with the foam cuffs. A syringe can be inserted through a port into the lumen to be used to withdraw the air from the interstices in the foam cuffs. When the air is withdrawn, the foam cuff retention means assume a contracted condition for insertion into the patient's urethra (50). The hypodermic can then be used to inject air into the interstices, thereby causing the retention means (12), (13) to expand to hold the incontinence device in position within the urethra (50). The valve (20) in this embodiment is a two-position ball valve that is manually conducted along a channel to alternatively close and open the exit end of the flexible tube (10). The ball (30) in the valve can be seated in recesses in the valve (20) to retain the ball (30) in the open position when urination is desired and in the open position when urination is completed.

Fig. 2

EP 0 265 207 A1

**Description**

TRANS-URETHRAL INCONTINENCE DEVICE

Background of the Invention

This invention relates to devices for controlling urinary incontinence, and in particular to in-dwelling trans-urethral devices.

Urinary incontinence is a long-recognized medical condition posing a serious and embarassing problem to those persons whose natural urethral valve or sphincter is no longer capable of controlling the flow of urine from the bladder. This problem can be the result of surgery, disease, neurological dysfunction, malformation of the urethral valve, and the natural physical deterioration accompanying advanced age. Solutions to the problem of urinary incontinence are many and are typically uncomfortable, unsanitary, inconvenient, offensive, inadequate, or unreliable.

Devices to control urinary incontinence include a pad of absorbent material to absorb all fluid that involuntarily escapes the bladder. Another method is to insert a catheter into the urinary tract with an exterior sealed collecting bag connected to the catheter tube. Penile clamps for human males are another management technique whereby the clamp exerts pressure against the urethra to prevent outflow of fluid.

Artificial urethral valves represent yet another solution to the problem of urinary incontinence. In this type of device, the function of the urethral valve, or sphincter, is assumed by a mechanical valve of a variety of designs. Kwan-Gett et al., U.S. Patent No. 3,768,102, discloses an implantable movable valve occluding means held in position by a spring calibrated to allow the valve to open when overcome by sufficient pressure from the bladder contents. The sprung valve remains open while under the normal flow of fluid from the bladder. Unlike Kwan-Gett, the device of the present invention is not implanted, requiring a surgical operation, but is removably in-dwelling. In addition, the present invention is manually operated to minimize the risk of accidental discharge as is possible with a spring valve mechanical device.

Isaacson, U.S. Patent No. 3,812,841, describes a magnetic valve positioned within the urethra by retention collars. An external magnetic field is required to open the sprung ball valve within the device. The apparatus of Isaacson is more sophisticated and, consequently, more susceptible to mechanical or operational failure than the present invention. Isaacson also required the user to carry a hand-held device to generate the magnetic field needed to operate the valve.

Other patents disclose similarly sophisticated mechanical and magnetic urethral valve devices, such as Osthagen et al., U.S. Patent No. 3,642,004; Osthagen et al., U.S. Patent No. 3,503,400; Trick, U.S. Patent No. 4,419,985.; and Cornwell, U.S. Patent No. 4,457,299.

The syringe unit of Broman, U.S. Patent No. 2,784,716, discloses a device having a two-position ball valve, wherein a ball is seated within a plastic sleeve. The ball is longitudinally movable from a closed position sealing a flow tube to an open position exposing an outflow orifice. The Broman device relates to a disposable enema bag which, with the ball valve, is held for use outside the body.

Summers, U.S. Patent No. 3,807,408, discloses a retention catheter comprising concentric shiftable tubes held in engagement with the interior wall of the bladder by deformable stretch. Relative axial shifting of the concentric tubes deforms the stretch from a collapsed configuration to an enlarged, pre-selected configuration to retain the catheter within the urethra and against the bladder sphincter. Miller, Jr. et al, Pat. No. 3,889,685, and Kamen et al., Pat. No. 3,640,282, each disclose a cuff comprised of a resilient member that maintains a normally expanded condition. The cuff in each invention can be contracted by means of a vacuum applied within the cuff, so that the device can be inserted into a trachea or blood vessel.

One object of the present invention is to provide a reliable device for incontinence control. Another object is to provide a device that has few movable parts and that is easily operable by the patient.

Yet another object of the present invention is to provide an incontinence control device that is not subject to mechanical failure or leakage. Other objects of this invention will become apparent to one skilled in the art through the accompanying disclosure and claims.

Summary of the Invention

One embodiment of the device of the present invention might include a urethra insertable in-dwelling incontinence device. There is provided a tube having at least one deformable retention means affixed to the exterior thereof. A valve means is situated at the end of the tube for opening and closing the end. The deformable retention means, in one embodiment, is a foam cuff having interconnecting interstices, and a deformable cover surrounding said cuff. The retention means is capable of assuming a contracted and a relatively enlarged condition when the interstices are evacuated or filled with a fluid, respectively. The valve means includes a first and a second channel, and a valve member situated to be conveyed along said first channel into recesses at either end of the first channel. The recesses are situated such that when the valve member is seated within a given recess the second channel is either opened or closed, thereby preventing or allowing fluid to exit through the valve means.

One embodiment of the method of the present invention might include a process for inserting a trans-urethral incontinence control device into a patient. The steps include providing an incontinence control device including a tube, a valve and a foam cuff, evacuating interstices in the foam cuff until it assumes a contracted shape, inserting the device into the urethra with the foam cuff in the bladder and the valve manually accessible to the patient at the exit end of the urethra, and introducing fluid into the interstices until the foam cuff assumes an expanded shape preventing travel of the incontinence control device within the urethra.

Brief Description of the Drawings

FIG. 1 is a side elevational view of one embodiment of the incontinence control device of the present invention.

FIG. 2 is a reduced side elevational view of the device of FIG. 1 showing it inserted within the urinary tract of a patient.

FIG. 3 is an enlarged end view of the device, taken at line 3-3 in FIG. 1 and viewed in the direction of the arrows.

FIG. 4 is an enlarged cross-sectional view of the device, taken at line 4-4 in FIG. 1 and viewed in the direction of the arrows.

FIG. 5 is an enlarged cross-sectional view of the device, taken at line 5-5 in FIG. 1 and viewed in the direction of the arrows, showing the retention means and lumen feed orifice.

FIG. 5A is an enlarged cross-sectional view of the device as in FIG. 5, showing the retention means in the enlarged and contracted conditions.

FIG. 6 is an enlarged fragmentary view of a portion of the foam cuff of the retention means shown in FIG. 5.

FIG. 7 is an enlarged longitudinal sectional view of the valve of the device of FIG. 1, shown with the ball valve member removed.

FIG. 8 is an enlarged longitudinal sectional view of the valve as in FIG. 8, showing the valve in the closed position.

FIG. 9 is an enlarged longitudinal sectional view of the valve as in FIG. 8, showing the valve in the open position.

FIG. 10 is a fragmentary side elevational view of an alternate embodiment of the present invention, showing a funnel as a retention means for the device.

FIG. 11 is a fragmentary side elevational view of an alternate embodiment of the present invention showing the longitudinal cross-sectional shapes of two deformable retention means.

FIG. 12 is a side cross-sectional view of a urinary incontinence device showing with a flexible pusher rod, used to convey the device into the urethra, inserted therein.

Description of the Preferred Embodiment

For the purposes of promoting an understanding of the principles of the invention, reference will now be made to the embodiments illustrated in the drawings and specific language will be used to describe the same. It will nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

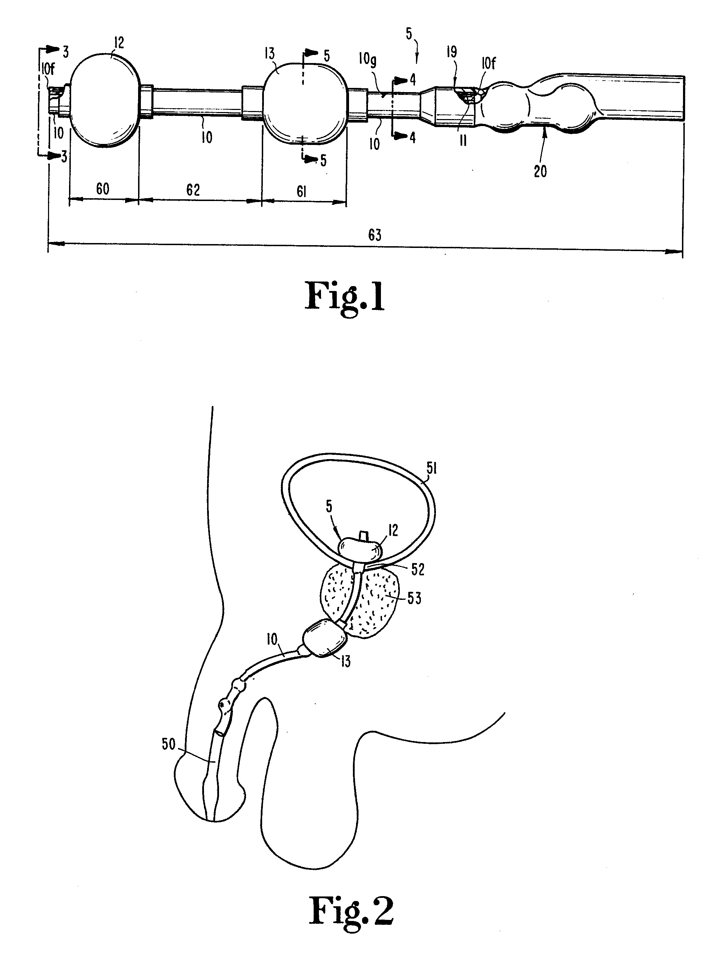

FIG. 1 illustrates one embodiment of the present invention. FIG. 2 is a sectional view of the patient's body showing a portion of this embodiment as it resides within the urinary tract of the patient. The incontinence control device 5 comprises a flexible tube 10 having a length approximately that of the urethra 50 of the patient in which the device is to be inserted. In this embodiment, two deformable retention means, 12 and 13, are fixed to the exterior surface of the tube. The distance between the retention means corresponds to the distance between the bladder sphincter 52 and the prostatic or bulbous urethra 53 of the patient. The retention means 12 is located near one end of the tube 10, and is situated inside the bladder 51 abutting the bladder sphincter when the incontinence control device is inserted in the patient. The second retention means 13 abuts the bulbous urethra. This arrangement of retention means prevents the incontinence control device 5 from travelling within the urinary tract, either upward into the bladder 51 or downward along the urethra 50.

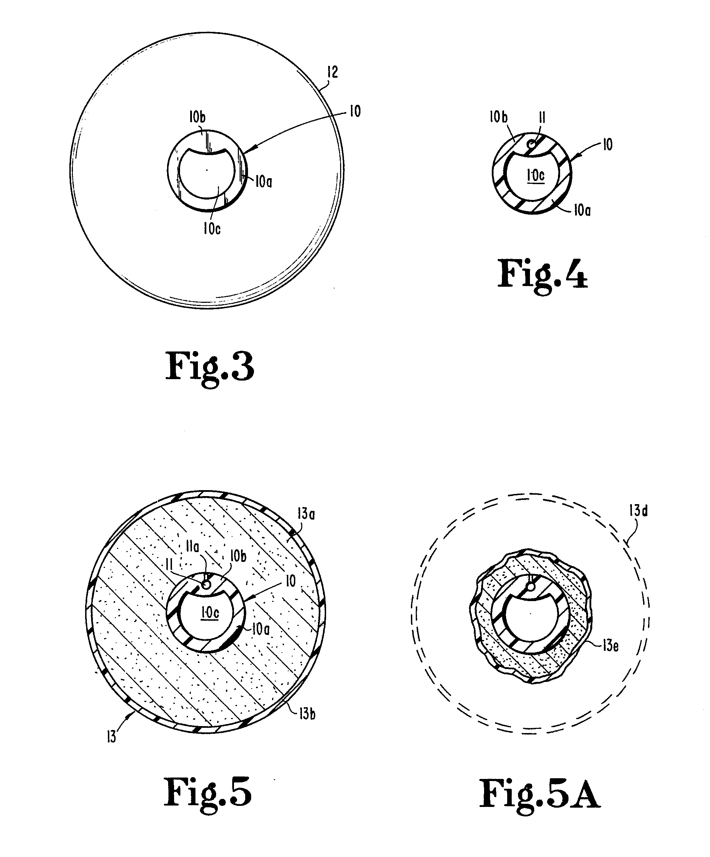

FIG. 3 is an end view of the segment of the tube 10 that resides within the bladder when the device is inserted within the patient. The tube has a flow channel 10c that extends along the entire length of the tube through which urine from the bladder flows to exit the body. The tube wall 10a has a thickened portion 10b that also extends along the entire length of the tube 10.

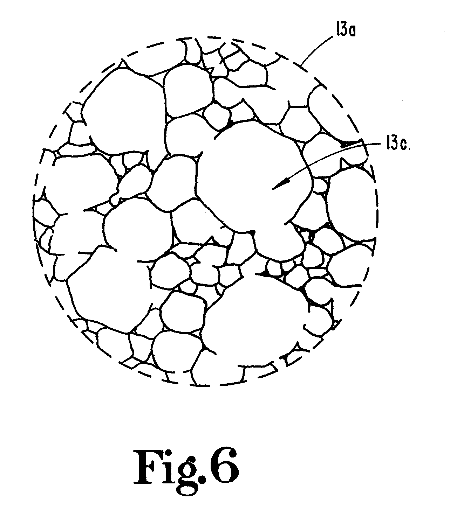

Referring to FIG. 4, it is seen that a lumen 11 is formed in the interior of the thickened portion 10b of the tube. The lumen 11 extends along the length of the tube but terminates at a plug 10f (FIG. 1) at each end of the tube, so that the lumen is sealed as shown in the end view of FIG. 3. At each of the retention means 12 and 13, the lumen 11 has a feed orifice 11a that communicates fluid from the interior of the lumen to the exterior of the tube 10. The retention means 12 and 13 surround the lumen feed orifice as shown in FIG. 5. The section view in FIG. 5 is typical at either retention means in this embodiment. The retention means 13 comprises a foam cuff 13a and a deformable cover 13b that surrounds and contains the foam cuff. The foam cuff comprises a plurality of interconnecting interstices, such as 13c in FIG. 6. Air or other fluid injected into the retention means through the lumen 11 and feed orifice 11a fills the interstices 13c, causing the foam cuff to expand to position 13d in FIG. 5A. Likewise, the air or other fluid can be withdrawn from the interstices, causing the foam cuff to contract against the tube to position 13e in FIG. 5A.

The tube 10 and deformable cover 13b are composed of a resilient material, such as a silicon material sold under the mark SILASTIC. The material can also be urethane or some other non-corrosive, resilient material suitable for prolonged non-irritating residence within the body. The foam cuffs are typically composed of a closed-cell polyethylene material. The deformable cover 13b is bonded onto the exterior of the tube 10 using a room temperature volatizing silicone compound to form an air-tight seal around the foam cuff 13a.

When the incontinence device is to be inserted into the patient's body, the air is withdrawn from the interstices 13c. A syringe (not shown) is inserted through an evacuation port 10g (FIG. 1) into the lumen 11. Air is withdrawn from the lumen, and, consequently, the retention means 12 and 13, into the syringe chamber as the syringe plunger is pulled through the syringe body. When the interstices 13c in the foam cuffs 13a of the retention means are sufficiently evacuated, the syringe is withdrawn from the port 10g. The elasticity of the tube material allows the port to be "self-sealing" so that the tube material constricts the port opening when the syringe is removed. Thus, the air-tight seal in the lumen is maintained and the retention means remains in the contracted condition while the incontinence control device is inserted into the patient's urethra.

Once the incontinence control device 5 is in position within the patient's body, as shown in FIG. 2, the syringe is again inserted through the evacuation port 10g. Air, or some other suitable fluid, is introduced from the syringe chamber into the lumen 11. The fluid flows through the lumen feed orifices 11a into the foam cuffs 13a at each of the retention means 12 and 13. The interstices 13c in the foam cuffs are filled by the fluid causing the cuffs to expand to the position 13d in FIG. 5A.

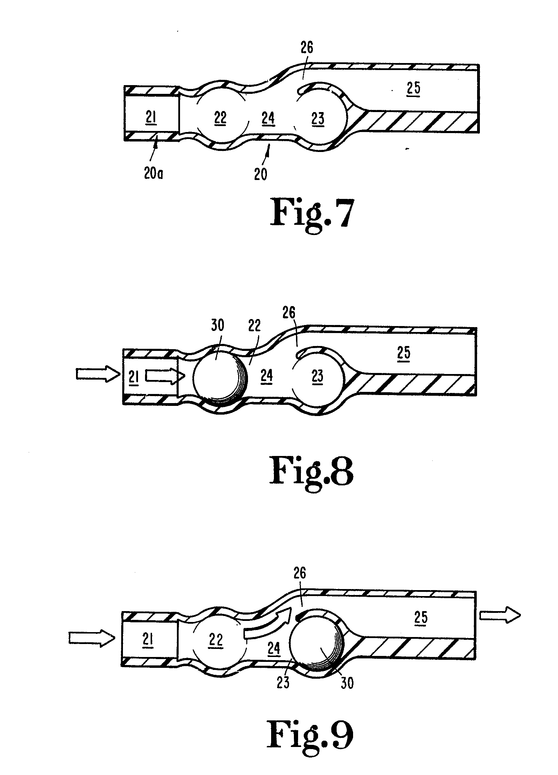

Referring again to FIG. 1, the incontinence control device 5 further comprises a valve at the exit end of the tube 10. In this embodiment of the invention, the valve 20 is manually-operable two-position ball-valve. It is recognized, however, that other valve devices are permissible to regulate the flow of urine from the incontinence control device. In this embodiment, the valve 20 is bonded onto the tube 10 at joint 19 using a room temperature volatizing silicone compound. The valve 20 is composed of a resilient material, such as SILASTIC. The valve material should be sufficiently resilient to withstand frequent manipulation without tearing or losing elasticity. The valve, as illustrated in FIG. 7, is molded as one piece. The inner diameter of the inlet portion 21 contacts the outer diameter of the flexible tube 10 to form the joint 19 (FIG. 1). The first channel 24 has a first recess 22 and a second recess 23 formed at its opposite ends. The second channel 25 is situated to communicate fluid from the first channel 24, through the opening 26, and ultimately to the exterior of the patient's body.

A valve member 30, in this instance a plastic ball, is situated in the first channel 24 and travels between the first and second recesses, 22 and 23, respectively. The valve member can also be plastic, such as neoprene, or some other corrosion resistant material embodiment, the ball is solid and has a diameter of 0.25 inches.

Each of the recesses are configured to elastically conform to a substantial portion of the ball 30 to retain the ball within the recess until manually dislodged by the patient. In FIG. 8, the valve is shown in the closed position in which the ball 30 is located in the first recess 22. The fluid flowing into the valve, represented by the large arrows in FIG. 8, cannot flow around the ball 30 as the ball and the valve wall 20a form an elastic seal. In the open position of FIG. 9, the ball 30 is moved to the second recess 23, thereby exposing the path, represented by the large arrows, through the first channel 24, through the opening 26 and to the second channel 25. In use by the patient, the valve 20 will normally be in a closed position with the ball 30 located in the first recess 22. The elastic seal formed by the ball 30 and the valve wall 20a is sufficiently strong to withstand the fluid pressure exerted by the urine in a full bladder. When the bladder is full, the patient can open the valve 20 by exterior manipulation of the valve to unseat the ball from the first recess 22 and transfer the ball to the second recess 23. The ball will remain trapped in the second recess during urination, as fluid passes through the first and second channels, 24 and 25, respectively. The opening 26 between the first and second channels has an effective diameter significantly smaller than the diameter of the ball 30. Thus, the ball is unlikely to exit the valve through the opening 26 and second channel 25 unless the valve is severely distended by excessive manipulation.

In the above mentioned one specific embodiment, the tube flow channel 10c has a diameter of 0.125 inches, while the tube outer diameter is 0.187 inches. The first retention means 12 typically has an expanded diameter of 1.00 inches and a width (the dimension 60) of 0.38 inches. The second retention means 13 has a diameter of 0.50 inches and a length (the dimension 61) of 0.60 inches. The diameter of the lumen 11 is 0.015 inches. In this specific embodiment, the distance 62 between the first and second retention means is 1.12 inches. The combined length (the dimension 63) of the tube 10 and valve 20 is typically between 5.7 and 5.9 inches. The dimensions 62 and 63 are sized to fit the device within a particular patient. In the case of the male, the entire incontinence control device is contained within the urethra, the valve residing adjacent the end of the penis. Thus, the valve is manipulated through the wall of skin of the penis.

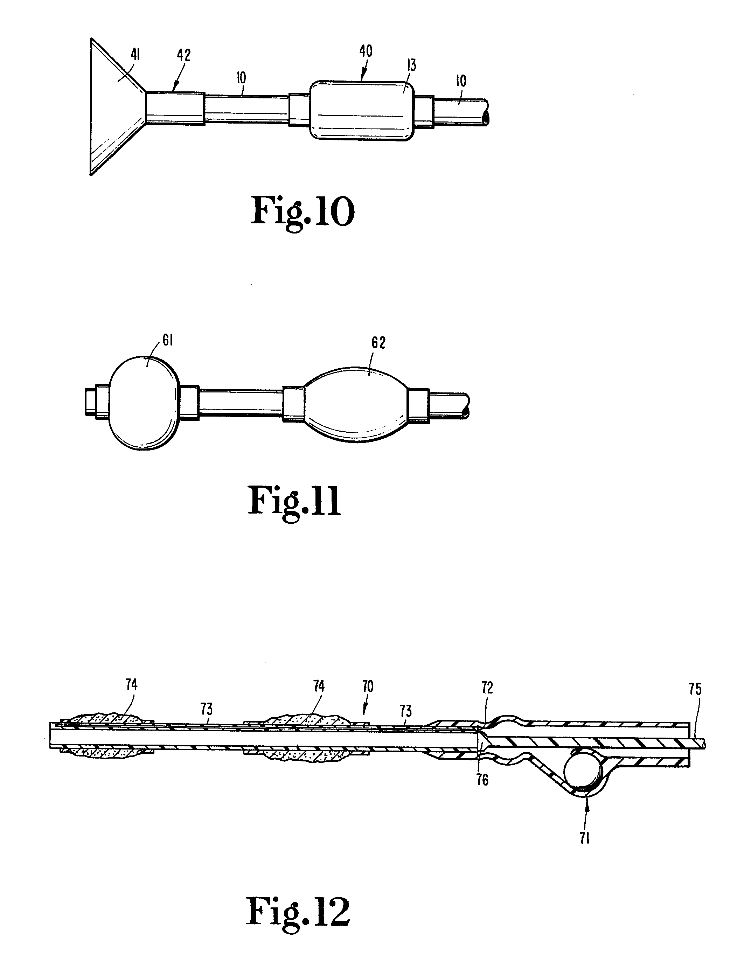

In another embodiment of the present invention, the first retention means is replaced by a flexible funnel 41, illustrated in FIG. 10. In some instances, a patient may have a urethra that is so enlarged that the foam cuff of the prior embodiment is insufficient to keep the incontinence control device from migrating out of the urethra. In these cases, the flexible funnel 41 is mounted in the bladder abutting the bladder sphincter. The configuration of the funnel as compared to the foam cuff provides a greater area of contact with the inner surface of the bladder, thereby improving the retentive characteristics of the device. The stem 42 is bonded onto the tube 10 to seal the funnel onto the tube. In one version of the incontinence control device 40 of this embodiment, the second retention means 13 is included, and positioned for seating adjacent the bulbous urethra in the same position as described above with regard to FIG. 1. In another version, the second retention means is eliminated, the incontinence control device being kept in position within the urethra solely by the flexible funnel 41.

In other versions of the above embodiments, the foam cuffs can have various longitudinal cross-sectional shapes. The foam cuffs can be generally circular in longitudinal cross-section, as in cuff 61 in FIG. 11, or oval, as in cuff 62. The foam cuffs can also be rectangular in cross-section, as in cuff 13 in FIG. 10. The shape of the foam cuff is dependent on its location within the urinary tract and upon the physical features of the patient's bladder, sphincter, bulbous urethra and urethra.

FIG. 12 illustrates another embodiment of the present invention, wherein a flexible pusher rod 75 is used to insert the incontinence control device 70 into the urethra of the patient. The pusher rod 75 is inserted through the valve 71 until the head 76 at the end of the pusher rod contacts the end surface 72 of the tube 73. The free end of the flexible pusher rod extends beyond the end of the valve 71 and beyond the end of the urethra so that it is accessible outside the body of the patient. The incontinence control device 70, shown with the retention cuffs 74 in the contracted condition, is advanced into the urethra by pushing on the pusher rod 75. When the device is properly seated, the pusher rod is withdrawn from the valve 71.

It is apparent from the foregoing description that the present invention offers the advantage of an in-dwelling, rather than an implanted, device, so that the device may be inserted and removed without the necessity of a surgical procedure and the pain and discomfort to the patient that necessarily accompanies surgery. Further, the present invention eliminates the need for a sophisticated external device to operate the valve, or for a complicated and cumbersome method for operating the valve.

While the invention has been illustrated and described in detail in the drawings and foregoing description, the same is to be considered as illustrative and not restrictive in character, it being understood that only the preferred embodiments have been shown and described and that all changes and modifications that come within the spirit of the invention are desired to be protected.

## Claims

1. A urethra insertable in-dwelling incontinence control device, comprising:
a flexible tube adapted to be inserted in a urinary tract of a patient, said tube having a first end and a second end;
at least one deformable retention means for restraining said tube within the urinary tract, said retention means being capable of assuming a contracted condition and also being capable of assuming a relatively enlarged condition; and
valve means for opening and closing said second end of the flexible tube, said valve means being affixed to the second end of the flexible tube and adapted to be inserted in said urinary tract.

2. The device of claim 1, wherein said deformable retention means comprises:
a foam cuff surrounding said flexible tube and having a plurality of interconnecting interstices, said foam cuff assuming said contracted condition when said interstices are evacuated and said cuff assuming said enlarged condition when the interstices are filled with a fluid;
a deformable cover surrounding said cuff and engaged with the exterior of the flexible tube to form an air-tight seal around the foam cuff; and
means for introducing fluid into and evacuating said interstices while preserving the air-tight seal during and after said introduction or evacuation.

3. The device of claim 1 or 2, wherein said valve means comprises;
a valve member;
a first channel, having a first recess at one end of said first channel in which said valve member is seated to prevent communication of fluid from said second end of the flexible tube when the valve is in a closed position, and, further, having a second recess at the other end of the first channel in which the valve member is seated when the valve is in an open position; and
a second channel situated to communicate fluid from the flexible tube when said valve member is seated in said second recess.

4. The device of claim 3, wherein;
said valve member has a smooth spherical surface;
each of said first and second recesses is shaped to accept said valve member and to retain the valve member within said recess; and
said first recess is further shaped to elastically conform to the valve member to restrict fluid flow between said valve member and the interior surface of the first recess.

5. The device of any preceding claim, wherein:
said valve means is manually operable; and
said valve means is adapted to be situated within the penis adjacent the exit end of the urethra.

6. The device of any one of claims 3, 4 or 5, wherein;
said first channel is composed of a resilient material so that the exterior of the channel can be manipulated in such a manner to dislodge said valve member from and convey the valve member between each of said recesses.

7. The device of any one of claim 3, 4, 5 or 6 wherein:
said first and second channels are integrally formed into said second end of the flexible tube.

8. The device of any one of claims 2 to 7, wherein said means for introducing fluid comprises:
a flexible lumen extending substantially along the length of said flexible tube, said lumen extending to and communicating with said foam cuff; and
a plug at each end of said lumen.

9. The device of claim 8, wherein:
said lumen is integrally formed along the interior of said flexible tube.

10. The device of claim 9, further comprising:

a port communicating through said tube with one end of said lumen, said port being adapted for having a syringe inserted therethrough, and being capable of substantially closing when the syringe is removed from the port.

11. The device of any one of claims 2 to 10, comprising:

a first retention means situated at said first end of the flexible tube; and

a second deformable retention means situated in fixed relationship with said first retention means and adapted to be situated at a bulbous urethra when said first retention means is situated at a bladder sphincter.

12. The device of any preceding claim, comprising:

a first retention means integrally formed into said first end of the flexible tube; and

said first retention means comprising a flexible cup having a generally funnel shape adapted to conform to the interior surface of a bladder of the patient when said cup is situated at a bladder sphincter.

13. The device of claim 12, further comprising a second deformable retention means situated in fixed relationship with said first retention means and adapted to be situated at a bulbous urethra when said first retention means is situated at the bladder sphincter.

14. The device of any preceding claim, comprising:

a first retention means situated at said first end of the flexible tube; and

said first retention means is substantially circular in longitudinal cross-section.

15. The device of any one of claims 2 to 14, wherein;

said second deformable retention means is substantially oval in longitudinal cross-section.

16. The device of any one of claims 1 to 14, wherein;

said second deformable retention means is substantially rectangular in longitudinal cross-section.

17. A method for inserting a trans-urethral incontinence device into a patient, comprising the steps of

1) providing an incontinence control device including a flexible tube having a deformable retention element situated at one end of said tube, said retention element comprising a foam cuff having interconnecting interstices and a deformable cover surrounding said cuff and engaged with said tube to form an air-tight seal about said foam cuff, and a valve regulating fluid flow through the tube;

2) evacuating said interstices in said foam cuff until said retention element assumes a contracted condition;

3) inserting the end of said flexible tube having the deformable retention element into the urethra;

4) conveying said tube into the urethra until the retention element is extended into the bladder, while the valve is manually accessible to the patient at the exit end of the urethra; and

5) introducing fluid into the interstices until the foam cuff assumed an expanded condition such that the retention element is restrained against the bladder sphincter to prevent travel of the incontinence control device within the urethra.

18. The method of claim 17, wherein the step of conveying said tube into the urethra further comprises the steps of:

contacting said flexible tube with a flexible pusher rod, said pusher rod at all times having a portion of the rod external to the body of the patient; and

pushing said pusher rod so that the rod reacts against the flexible tube to convey said tube into the urethra.

19. A method for inserting a trans-urethral incontinence control device into a patient, comprising the steps of:

1) providing an incontinence control device including a flexible tube having a first deformable retention element located at the end of said tube and a second deformable retention element in spaced relation with said first retention element, each of said retention elements comprising a foam cuff having interconnecting interstices and a deformable cover surrounding said cuff and engaged with said tube to form an air-tight seal about said foam cuff, and a valve regulating fluid flow through the tube;

2) evacuating said interstices in said foam cuff in each of said deformable retention elements until each of the retention elements assumes a contracted condition;

3) inserting the end of said flexible tube having the first deformable retention element into the urethra;

4) conveying said tube into the urethra until the first retention element is extended into the bladder, while the second retention element is situated at the bulbous urethra, and while the valve is manually accessible to the patient at the exit end of the urethra; and

5) introducing fluid into the interstices until the foam cuff in each of the retention elements assumes an expanded condition such that the first retention element is restrained against the bladder sphincter and the second retention element is restrained against the bulbous urethra to prevent travel of the incontinence control device within the urethra.

20. The method of claim 19, wherein the step of conveying said tube into the urethra further comprises the steps of;

contacting said flexible tube with a flexible pusher rod, said pusher rod at all times having a portion of the rod external to the body of the patient; and

pushing said pusher rod so that the rod reacts against the flexible tube to convey said tube into the urethra.

21. A method for operating an incontinence control valve, comprising the steps of:

1) providing an incontinence control device comprising a two-position valve situated at the exit end of the urethra and accessible by the patient in whom the device is inserted, said two-position having a channel with a first recess at one end of said channel in which a ball is seated when the valve is in a normally closed position, and with a second recess at the other end of said channel in which the ball is seated when the valve is in an open position;

2) manually manipulating the exterior of said two-position valve to unseat the ball from said first recess in the normally closed position;

3) manually manipulating the exterior of the valve to convey the ball along said channel from the first recess toward said second recess;

4) manually manipulating the exterior of the valve to seat the ball within said second recess to place the valve in the open position allowing fluid to flow from the incontinence control device during urination;

5) manually manipulating the exterior of the valve to unseat the ball from the second recess, once urination is complete;

6) manually manipulating the exterior of the valve ot convey the ball along the channel from the second recess toward the first recess; and

7) manually manipulating the exterior of the valve to seat the ball within the first recess to place the valve in the normally closed position.

0265207

Fig.1

Fig.2

0265207

Fig.3

Fig.4

Fig.5

Fig.5A

0265207

Fig.6

0265207

Fig.7

Fig.8

Fig.9

**Fig.10**

**Fig.11**

**Fig.12**

**European Patent Office**

**PARTIAL EUROPEAN SEARCH REPORT**
which under Rule 45 of the European Patent Convention
shall be considered, for the purposes of subsequent
proceedings, as the European search report

Application number

EP 87 30 9214

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | US - A - 3 812 841 (L. ISAACSON) <br> * Figures 1,7; column 2, line 45 - column 3, line 62; column 5, lines 34-41 * | 1,11, 14 | A 61 F 2/48 |
| A | | 2 | |
| A,D | -- <br> US - A - 2 784 716 (C. BROMAN) <br> * Figure 3; column 3, lines 4-10 * | 3,4 | |
| | -------------------- | | |

TECHNICAL FIELDS
SEARCHED (Int. Cl.4)

A 61 F
A 61 M

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-16
Claims searched incompletely:
Claims not searched: 17-21
Reason for the limitation of the search:

Method for treatment of the human or
animal body by surgery or therapy
(see art. 52(4) of the European Patent
Convention).

| Place of search | Date of completion of the search | Examiner |
|---|---|---|
| The Hague | 26-01-1988 | NEILL |